# EUROPÄISCHE PATENTSCHRIFT

(11) **EP 0 566 150 B1**
(45) Veröffentlichungstag und Bekanntmachung des Hinweises auf die Patenterteilung: **23.10.1996**
(21) Anmeldenummer: 93106257.4
(22) Anmeldetag: 16.04.1993
(51) Int. Cl.: A61F 13/15, B29C 70/00, B29C 65/34, B29L 31/24

(54) **Verfahren zur Herstellung einer hautberührenden Decklage einer Wegwerfwindel mit einer elastischen Öffnung**
Process for the production of a skin contact top sheet for a disposable diaper with an elastic opening
Procédé pour fabriquer une feuille supérieure en contact avec la peau du bébé dans une couche à jeter pourvu d'une ouverture élastique

(30) Priorität: 17.04.1992 JP 124284/92
(43) Veröffentlichungstag der Anmeldung: 20.10.1993
(73) Patentinhaber: UNI-CHARM CORPORATION, Kawanoe-shi Ehime-ken (JP)
(72) Erfinder: Tanji, Hiroyuki, Kawanoe-shi, Ehime-ken (JP); Wada, Ichiro, Kawanoe-shi, Ehime-ken (JP); Ono, Yoshio, Kawanoe-shi, Ehime-ken (JP); Soga, Hiroyuki, Kawanoe-shi, Ehime-ken (JP)
(74) Vertreter: Sperling, Rüdiger, Dipl.-Ing.

(56) Entgegenhaltungen:
- EP-A- 0 200 482
- EP-A- 0 391 476
- EP-A- 0 486 006
- FR-A- 2 668 364
- US-A- 4 662 877

## Beschreibung

### DER ERFINDUNG ZUGRUNDE LIEGENDER STAND DER TECHNIK

Die Erfindung betrifft ein Verfahren zur Herstellung einer hautberührenden Decklage einer Wegwerfwindel mit einer elastischen Öffnung zur zuverlässigen Einführung von Ausscheidungen.

Die japanische Gebrauchsmusteranmeldung Amtsblatt-Nr. 1974-120439 zeigt eine Windeleinlage auf, die eine Decklage aufweist, die in ihrem Mittelbereich mit einer sich in Längsrichtung weiter als in Querrichtung der Decklage erstreckenden Öffnung versehen ist, wobei die Öffnung entlang ihrem Umfangsrand mit einem in Längsrichtung dehnbaren elastischem Element versehen ist, um so eine geschlossene ringförmige elastische Linie zu bilden. Die japanische Patentanmeldung Amtsblatt-Nr. 1986-41304 zeigt ebenfalls eine Wegwerfwindel auf, die mit einer Decklage versehen ist, in deren Mittelbereich eine Öffnung ausgebildet ist, die in Längsrichtung der Decklage sich weiter erstreckt als in Querrichtung derselben, wobei die Öffnung entlang ihren seitlich gegenüber liegenden Seitenrändern jeweils mit elastischen Elementen versehen ist.

Bei dieser Windeleinlage und Windel, die beide mit Öffnungen versehen sind, fließen Ausscheidungen durch die Öffnung in Taschen, die zwischen der Decklage und einer getrennt unter der erstgenannten Decklage vorgesehenen Decklage gebildet ist, und werden darin gehalten, was es ermöglicht, die Gefahr zu vermeiden oder wenigstens zu verringern, daß die Haut des Trägers mit den über die mit der Haut in Berührung stehende oberste Decklage verteilten Ausscheidungen verschmiert wird, was dem Träger ein unangenehmes Gefühl vermittelt und sogar zum Ausbruch von Hautkrankheiten beim Träger führen kann.

Die vorstehend genannte japanische Gebrauchsmusteranmeldung Amtsblatt-Nr. 1974-120439 und die japanische Patentanmeldung Amtsblatt-Nr. 1986-41304 zeigen beide kein Verfahren zur Ausbildung der hautberührenden Decklage mit der Öffnung oder ein Verfahren zum Anbringen des elastischen Elementes um die Öffnung auf. Gemäß ersterem Dokument wird angenommen, daß der gesamte Umfangsrand der Öffnung mit einem elastischen Element in endloser Ausführung versehen wird und anschließend das elastische Element mit dem Umfangsabschnitt der Öffnung bedeckt wird. In der Beschreibung findet sich jedoch kein bestimmtes Verfahren. Angesichts der Tatsache, daß sich ersteres Dokument auf eine wiederverwendbare Windeleinlage bezieht, werden in dem Verfahren wahrscheinlich Schneid- und Nähtechniken bekannter Art verwendet. Gemäß letzterem Dokument wird der Mittelteil der Lage ausgeschnitten, um die Öffnung zu bilden, und die beiden Seitenränder der Öffnung werden mit den jeweiligen elastischen Elementen versehen, die unter Verwendung von Klebstoff mit den Rückseiten der jeweiligen Seitenränder verklebt werden. Demzufolge ist die Windel gemäß letzterem Dokument nicht nur unansehnlich, sondern auch hinsichtlich der erwünschten Festigkeit entlang dem Umfangsrand der Öffnung von mangelhafter Qualität, da die elastischen Elemente überhaupt nicht bedeckt sind, obwohl sie an der Rückseite der Lage vorgesehen sind. Darüber hinaus führt eine derartige Anordnung nicht zur Bildung einer ordentlich gleichmäßigen Raffung entlang dem gesamten Umfangsrand der Öffnung und daher kann es nicht erwartet werden, daß der gesamte Umfangsrand der Öffnung beim Tragen dicht an der Haut des Trägers anliegt.

Es ist eine wesentliche Aufgabe der Erfindung, eine hautberührende Decklage einer Wegwerfwindel mit einer Öffnung aufzuzeigen, deren gesamter Umfangsrand eine gewünschte Festigkeit aufweist und elastische Elemente enthält, die mit der Decklage bedeckt sind.

### BESCHREIBUNG DER ERFINDUNG

Gemäß der Erfindung wird vorstehend dargelegte Aufgabe durch ein Verfahren zur Bildung einer hautberührenden Decklage einer Wegwerfwindel mit einer elastischen Öffnung gelöst, wobei das Verfahren die Schritte umfaßt:
A. Zuführen eines endlosen, gedehnt gehaltenen elastischen Elementes auf eine endlose Bahn in Längsrichtung derselben, so daß das endlose elastische Element eine in Wellen verlaufende Linie beschreibt, die wechselweise auf einen ersten Seitenrand und anschließend auf einen zweiten Seitenrand der endlosen Bahn, der dem ersten Rand gegenüber liegt, zu gekrümmt ist, um so Wellenkämme und relativ breite Wellentäler jeweils zu bilden, und Verkleben des endlosen elastischen Elementes mit der Bahn unter Verwendung von Klebstoff, um eine erste endlose Verbundbahn zu erhalten;
B. Falten eines Bereiches der ersten endlosen Verbundbahn, der sich in unmittelbarer Nähe des ersten Seitenrandes derselben erstreckt, entlang einer in Längsrichtung in einer Höhe zwischen dem ersten Seitenrand und den Tälern verlaufenden Faltlinie, so daß die jeweiligen Kämme wenigstens teilweise innerhalb eines Bereiches zwischen der Faltlinie und dem ersten Seitenrand enthalten sind, gefolgt vom Verkleben des gefalteten Bereiches mit dem nicht gefalteten Bereich der ersten endlosen Verbundbahn, um eine zweite endlose Verbundbahn zu erhalten, die das endlose elastische Element umfaßt, das mit der endlosen Bahn bedeckt ist;
C. Abschneiden der Bereiche der zweiten endlosen Verbundbahn, die von den jeweiligen Kämmen, Tälern und der Faltlinie umgeben sind, um eine dritte endlose Verbundbahn zu erhalten, die somit mit Ausschnitten versehen ist, die zur Begrenzung der Hälften der jeweiligen Öffnungen bestimmt sind;
D. Nebeneinanderlegen von zwei derartigen dritten Verbundbahnen, wobei jeweils zwei zueinandergehörige Ausschnitte in einer in Querrichtung symmetrischen Beziehung einander gegenüberliegend miteinander fluchtend ausgerichtet sind, Auflegen dieser Bahnen auf eine Decklage einer endlosen Windelbahn, die eine Vielzahl von einzelnen Windeln umfaßt, so daß jeweils zwei Randabschnitte der jeweiligen dritten endlosen Verbundbahnen, die sich in Längsrichtung über jeweils zwei beidseits gegenüberliegende Kämme erstrecken, einander überlappen, und Verkleben der Bahnen entlang den äußeren Seitenrändern mit der Decklage der endlosen Windelbahn; und
E. Schneiden der endlosen Windelbahn, mit der nun die beiden dritten endlosen Verbundbahnen verklebt sind, in Querrichtung entlang Linien, die vertikal die Kämme halbieren, um die einzelnen Windeln zu erhalten.

Vorzugsweise umfaßt Schritt E weiter einen Schritt des Verklebens von jeweils zwei Randbereichen der jeweiligen dritten endlosen Verbundbahnen, die sich in Längsrichtung über jeweils zwei beidseits gegenüberliegende Kämme erstrecken, in einander überlappender Beziehung.

### KURZE BESCHREIBUNG DER ZEICHNUNG

Die Erfindung wird nun anhand eines Beispiels unter Bezug auf die beiliegenden Figuren erläutert, wobei
- Fig. 1: eine perspektivische Ansicht der Innenseite einer Wegwerfwindel zeigt die mit einer nach einem erfindungsgemäßen Verfahren ausgebildeten, hautberührenden Decklage versehen ist;
- Fig. 2: eine Schnittdarstellung entlang einer Linie 2-2 in Fig. 1 zeigt;
- Fig. 3: eine schematische Draufsicht zur Erläuterung der Art und Weise, in der das endlose elastische Element auf der endlosen Bahn als Ausgangsmaterial für die hautberührende Decklage angebracht wird, um eine erste endlose Verbundbahn zu bilden, zeigt;
- Fig. 4: eine schematische Draufsicht zur Erläuterung der Art und Weise, in der die endlose Bahn teilweise umgefaltet wird, um das endlose elastische Element zu bedecken, um so eine zweite endlose Verbundbahn zu bilden, gefolgt vom Ausschneiden der Abschnitte, die vom elastischen Element umgeben werden, um so eine dritte endlose Verbundbahn zu bilden, zeigt; und
- Fig. 5: eine schematische Draufsicht zur Erläuterung der Art und Weise, in der zwei derartiger dritter endloser Verbundbahnen in einander überlappender Beziehung nebeneinander gelegt werden, zeigt.

### BEVORZUGTE AUSFÜHRUNGSFORMEN DER ERFINDUNG

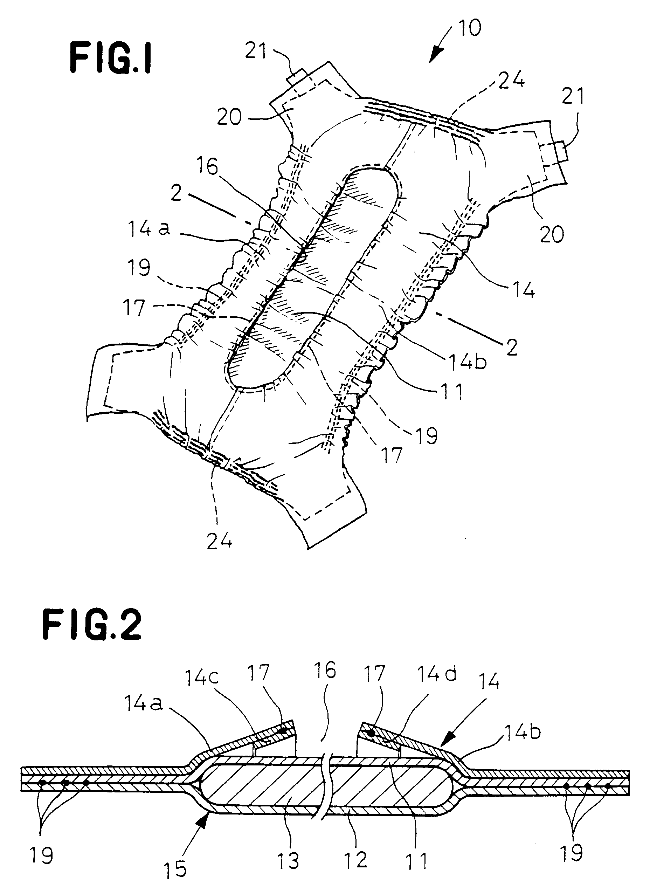

Wie Fig. 1 und 2 zeigen, umfaßt eine Windel 10 eine flüssigkeitsdurchlässige Decklage 11, eine flüssigkeitsundurchlässige Außenlage 12, einen flüssigkeitsabsorbierenden Kern 13, der zwischen diese gelegt ist, und eine flüssigkeitsbeständige, hautberührende Decklage 14. Im Mittelbereich der hautberührenden Decklage 14 ist eine Öffnung 16 ausgebildet, die in Längsrichtung größer ist als in Querrichtung dieser Lage 14 und deren in Längsrichtung gegenüberliegende Enden jeweils Kreisbögen beschreiben. Die Öffnung 16 kann wenigstens innerhalb des Schrittbereiches ausgebildet sein.

Die hautberührende Decklage 14 umfaßt eigentlich zwei Lagenelemente 14a, 14b, die einander seitlich überlappen und jeweils einander gegenüberliegende innere Seitenränder aufweisen, die mit Ausschnitten versehen sind, so daß jeweils zwei einander gegenüberliegende Ausschnitte jeweils die Öffnung 16 bilden. Verstärkungslagenelemente 14c, 14d, die aus demselben Material wie die Lagenelemente 14a, 14b hergestellt sind, werden mit den Rändern der jeweiligen Ausschnitte verklebt. Zwischen den Lagenelementen 14a, 14b und den Verstärkungslagenelementen 14c, 14d sind entlang den Rändern der jeweiligen Ausschnitte in Längsrichtung dehnbare elastische Elemente 17 unter Verwendung von Heißschmelzkleber (nicht dargestellt) angebracht. Die Längenabschnitte der inneren Ränder, die nach der Ausbildung der Ausschnitte in überlappender Beziehung bleiben, werden mit Heißschmelzkleber (nicht dargestellt) verbunden.

Die beiden elastischen Elemente 17 sind voneinander unabhängig im Gegensatz zu dem einzelnen endlosen elastischen Element, das einen endlosen Ring bildet, wie es in der vorstehend erwähnten Windeleinlage nach dem Stand der Technik verwendet wird. Zusätzlich sind die inneren Seitenränder der Lagenelemente 14a, 14b, die sich im Bereich der in Längsrichtung gegenüberliegenden Enden überlappen, unter Verwendung von Heißschmelzkleber miteinander verklebt. Eine derartige Anordnung erlaubt es, daß die hautberührende Decklage 14 leicht entlang den überlappenden Längenabschnitten der inneren Seitenränder abgerissen werden kann, wenn es erwünscht ist, beinahe die gesamte Oberfläche der unter der hautberührenden Decklage 14 liegenden Decklage freizulegen und dadurch feste Ausscheidungen, die zwischen der hautberührenden Dechlage 14 und der Decklage 11 befindlich sind, abzukratzen. Während die überlappenden inneren Seitenränder unter Verwendung des Klebers miteinander verklebt werden, ist die Klebekraft eines zum Verbinden derartiger Windeln verwendeten Heißschmelzklebers allgemein auch nach dem Aushärten nicht ausreichend, um das Abreißen zu erschweren.

Flüssige Ausscheidungen werden durch die Öffnung 16 auf die Decklage 11 geführt, durchdringen diese Decklage 11 und werden vom Kern 13 absorbiert, während feste Ausscheidungen in die Taschen eingeführt werden, die zwischen der Decklage 11 und der hautberührenden Decklage 14 gebildet sind. Derartige feste Ausscheidungen können von der Decklage 11 in einem gewünschten Ausmaß abgekratzt werden, wenn die hautberührende Decklage 14 von den in Längsrichtung gegenüberliegenden Enden der Öffnung 16 abgerissen wurde.

Zwischen den seitlich gegenüberliegenden Rändern der Decklage 11 und den seitlich gegenüberliegenden Rändern der Außenlage 12, die sich beide beiderseits des flüssigkeitsabsorbierenden Kernes 13 nach außen erstrecken, sind mehrere elastische Elemente 19, die jeweils wiederum mehrere elastische Fäden umfassen, in gedehntem Zustand unter Verwendung von Heißschmelzkleber (nicht dargestellt) jeweils angebracht, so daß sie in Längsrichtung der Lagen dehnbar sind und dicht um die Beine des Trägers anliegen. In ähnlicher Weise sind zwischen den in Längsrichtung gegenüberliegenden Enden der Decklage 11 und den zugehörigen Enden der Außenlage 12 mehrere elastische Elemente 24 vorgesehen, die jeweils wiederum mehrere elastische Fäden umfassen, so daß sie quer zu den Lagen dehnbar sind und dicht um die Hüfte des Trägers anliegen.

Die Decklage 11 kann aus Vließstoff, poröser Kunststoffolie oder ähnlichem, die Außenlage 12 aus Kunststoffolie, einer geschichteten Lage aus dieser Kunststoffolie und Vließstoff oder ähnlichem, und der flüssigkeitsabsorbierende Kern 13 aus einer Mischung aus Faserpulpe und hochabsorbierendem Polymerpulver oder ähnlichem hergestellt sein. Die hautberührende Decklage 14 und die Verstärkungslagenelemente 14c und 14d sind vorzugsweise aus wasserabstoßendem und hoch luftdurchlässigem Vließstoff hergestellt. Es sei angemerkt, daß in der Beschreibung der Erfindung der Begriff "flüssigkeitsbeständiges" Material sich auf Material bezieht, das einen ausreichenden Grad von wasserabweisenden Eigenschaften aufweist, um zu verhindern, daß flüssige Ausscheidungen ohne weiteres dieses durchdringen, wenn die Windel am Körper des Benutzers getragen wird.

Wie Fig. 1 zeigt, weist die Windel 10 jeweils zwei flügelähnliche Klappen 20 auf, die sich von den einander gegenüberliegenden Seitenrändern an der Hüftlinie jeweils nach außen erstrecken. Die freien Enden von Befestigungsbändern 21, die an den jeweiligen Rückseiten der flügelähnlichen Klappen 20 befestigt sind, können an der Außenlage 12 an der Vorderseite verklebt werden, um die Windel 10 um den Körper des Trägers aufzurichten.

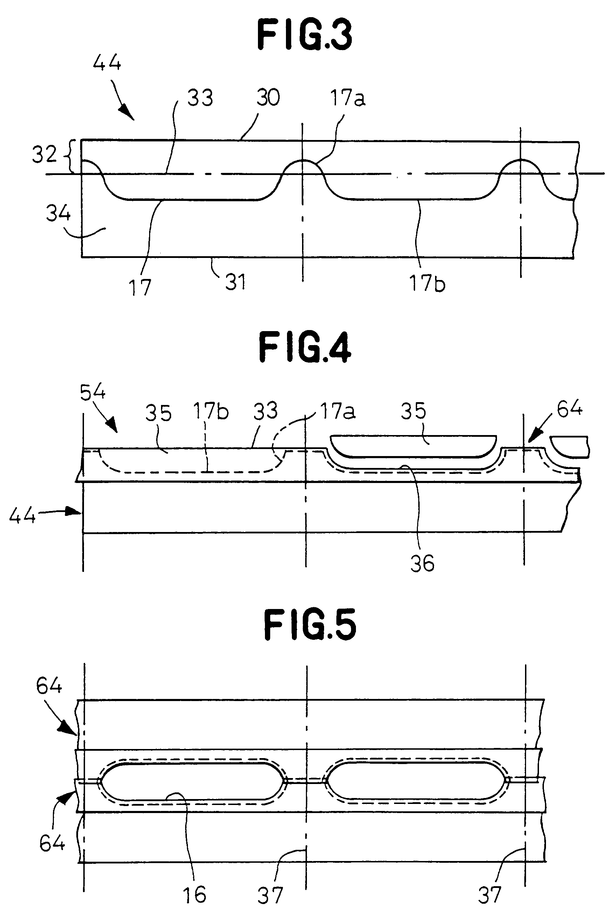

Nachfolgend wird ein Verfahren gemäß der Erfindung als Beispiel unter Bezug auf Fig. 3 bis 5 erläutert.

Wie Fig. 3 zeigt, wird ein gedehnt gehaltenes endloses elastisches Element 17 von nicht dargestellten Traversiermitteln auf eine Endlosbahn 34 in Längsrichtung derselben zugeführt, so daß das endlose elastische Element 17 eine in Wellen verlaufende Linie beschreibt, die wechselweise auf einen ersten Seitenrand 30 zu und anschließend auf einen zweiten Seitenrand 31 der endlosen Bahn 34, der dem ersten Seitenrand 30 gegenüberliegt, zu gekrümmt ist, so daß Wellenkämme 17a und relativ breite Wellentäler 17b jeweils ausgebildet werden, und mit der Bahn 34 unter Verwendung von Heißschmelzkleber (nicht dargestellt) verklebt wird, um eine erste endlose Verbundbahn 44 zu erhalten.

Wie Fig. 3 und 4 zeigt, wird ein Bereich 32 der ersten endlosen Verbundbahn 44, der sich an den ersten Seitenrand 30 derselben anschließend erstreckt, entlang einer Faltlinie 33, die sich in Längsrichtung zwischen dem ersten Seitenrand und den Wellentälern 17 erstreckt, gefaltet, so daß die jeweiligen Kämme 17a wenigstens teilweise innerhalb eines Bereiches enthalten sind, der zwischen der Faltlinie 33 und dem ersten Seitenrand 30 liegt. Dieser gefaltete Bereich 32 wird anschließend mit einem nicht gefalteten Bereich der ersten endlosen Verbundbahn 44 verklebt, um eine zweite endlose Verbundbahn 54 zu erhalten, die das endlose elastische Element 17 umfaßt, das mit der endlosen Bahn 34 bedeckt ist.

Bereiche 35 der zweiten endlosen Verbundbahn 54, die von den jeweiligen Kämmen 17a, Tälern 17b und der Faltlinie 33 umgeben sind, werden ausgeschnitten, um so eine dritte endlose Verbundbahn 64 zu erhalten, die somit mit Ausschnitten 36 versehen ist, die zur Bildung der Hälten von jeweiligen Öffnungen 16 bestimmt sind.

Wie Fig. 5 zeigt, werden zwei dritte endlose Verbundbahnen 64 nebeneinander so angeordnet, daß jeweils zwei einander zugeordnete Ausschnitte 36 einander gegenüberliegend und fluchtend in in Querrichtung symmetrischer Beziehung angeordnet sind, und auf eine Decklage einer endlosen Windelbahn (nicht dargestellt) aufgelegt, die eine Vielzahl von einzelnen Windeln umfaßt, wobei jeweils zwei Randbereiche der jeweiligen dritten endlosen Verbundbahnen 64, die sich in Längsrichtung über jeweils zwei beidseits einander gegenüberliegende Wellenkämme 17a der jeweiligen Bahnen 64 erstrecken, einander überlappen. Diese Bahnen 64 werden anschließend entlang ihren äußeren Seitenrändern auf die Decklage der endlosen Windelbahn aufgeklebt und die endlose Windelbahn, mit der nun die Bahnen 64 verklebt sind, wird in Querrichtung entlang Linien 37 geschnitten, die die jeweiligen Paare von beidseits gegenüberliegenden Wellenkämmen 17a in Hälften teilen, um so die einzelnen Windeln zu erhalten. Die überlappenden Randbereiche der jeweiligen dritten endlosen Verbundbahnen 64, die sich über die jeweiligen Paare von beidseits gegenüberliegenden Wellenkämmen 17a erstrecken, werden unter Verwendung von Heißschmelzkleber (nicht dargestellt) miteinander verklebt.

Es sei angemerkt, daß diese Randbereich nach Wunsch auch unverklebt belassen werden können.

Die endlose Windelbahn umfaßt eine Vielzahl von einzelnen Windeln 10 im wesentlichen in der in Fig. 1 dargestellten Form (ausgenommen das Fehlen der hautberührenden Decklage 14), die in Längsrichtung derselben aneinander anschließend miteinander verbunden sind. Das Verfahren zur Herstellung einer derartigen endlosen Windelbahn ist dem Durchschnittsfachmann aus verschiedenen Patentbeschreibungen bekannt, so daß daher die Details eines derartigen Verfahrens hier nicht beschrieben werden.

Während die Ausführungsform hinsichtlich sogenannter offener Windeln erläutert und beschrieben wurde, die die Befestigungsbänder zum Schließen der Hüftlinie um den Körper des Trägers verwenden, ist die Erfindung ebenfalls auf sogenannte Windeln des Höschentyps (einschließlich Erziehungshöschen) anwendbar, die eine endlose Hüftlinie aufweisen.

Wie aus vorstehender Beschreibung ohne weiteres ersichtlich ist, umfaßt die Windel gemäß der Erfindung eine hautberührende Decklage für jede einzelne Windel, die aus der dritten endlosen Verbundbahn geschnitten wird, die mit der Öffnung versehen ist, die entlang ihrem halben Umfang jeweils mit zwei voneinander unabhängigen Elementen versehen ist, um den gesamten Umfang elastisch zu gestalten, so daß die gleichförmig entlang dem gesamten Umfang der Öffnung ausgebildete Fältelung sich beim Tragen dicht an die Haut des Trägers anlegen kann und dadurch Ausscheidungen zuverlässig in die Öffnung eingeführt werden können. Desweiteren sind die jeweiligen elastischen Elemente mit gefalteten Bereichen bedeckt, die ebenfalls zur Verstärkung des Umfanges der Öffnung dienen, womit ein schönes Erscheinungsbild und eine hohe Festigkeit bei der Windel erzielt werden, die die hautberührende Decklage mit der darin ausgebildeten Öffnung umfaßt.

Gemäß der Erfindung umfaßt die hautberührende Decklage zwei Lagenelemente, die jeweils den Ausschnitt aufweisen, der zur Begrenzung einer Hälfte der Öffnung bestimmt ist, sowie die um diesen Ausschnitt angebrachten elastischen Elemente. Im einzelnen werden diese Lagenelemente seitlich nebeneinander so angeordnet, daß die jeweiligen Ausschnitte einander symmetrisch gegenüber liegen und miteinander fluchtend ausgerichtet sind, so daß sie die Öffnung in dem Mittelbereich der zusammengesetzten hautberührenden Decklage bilden. Mit einer derartigen Anordnung wird die Windel mit der hautberührenden Decklage verwirklicht, die so eingerichtet ist, daß sie leicht von den in Längsrichtung gegenüberliegenden Enden der Öffnung abgerissen werden kann, um feste Ausscheidungen, die auf der Decklage unter der hautberührenden Decklage befindlich sind, abzukratzen.

## Patentansprüche

1. Verfahren zur Bildung einer hautberührenden Decklage (14) einer Wegwerfwindel mit einer elastischen Öffnung (16), umfassend die Schritte:
A. Zuführen eines endlosen, gedehnt gehaltenen elastischen Elementes (17) auf eine endlose Bahn in Längsrichtung derselben, so daß das endlose elastische Element (17) eine in Wellen verlaufende Linie beschreibt, die wechselweise auf einen ersten Seitenrand (30) und anschließend auf einen zweiten Seitenrand (31) der endlosen Bahn, der dem ersten Rand (30) gegenüber liegt, zu gekrümmt ist, um so Wellenkämme (17a) und relativ breite Wellentäler (17b) jeweils zu bilden, und Verkleben des endlosen elastischen Elementes (17) mit der Bahn unter Verwendung von Klebstoff, um eine erste endlose Verbundbahn (44) zu erhalten;
B. Falten eines Bereiches, der ersten endlosen Verbundbahn (44), der sich in unmittelbarer Nähe des ersten Seitenrandes (31) derselben erstreckt, entlang einer in Längsrichtung in einer Höhe zwischen dem ersten Seitenrand (31) und den Tälern (17b) verlaufenden Faltlinie (33), so daß die jeweiligen Kämme (17a) wenigstens teilweise innerhalb eines Bereiches zwischen der Faltlinie (33) und dem ersten Seitenrand (31) enthalten sind, gefolgt vom Verkleben des gefalteten Bereiches (32) mit dem nicht gefalteten Bereich (34) der ersten endlosen Verbundbahn, um eine zweite endlose Verbundbahn (64) zu erhalten, die das endlose elastische Element (17) umfaßt, daß mit der endlosen Bahn bedeckt ist;
C. Abschneiden der Bereiche (35) der zweiten endlosen Verbundbahn (54), die von den jeweiligen Kämmen (17a), Tälern (17b) und der Faltlinie (33) umgeben sind, um eine dritte endlose Verbundbahn (64) zu erhalten, die somit mit Ausschnitten (36) ausgebildet ist, die zur Begrenzung der Hälfte der jeweiligen Öffnungen (16) bestimmt sind;
D. Nebeneinanderlegen von zwei derartigen dritten Verbundbahnen (54), wobei jeweils zwei zueinandergehörige Ausschnitte (36) in einer in Querrichtung symmetrischen Beziehung einander gegenüberliegend miteinander fluchtend ausgerichtet sind, Auflegen dieser Bahnen (64) auf einer Decklage (11) einer endlosen Windelbahn, die eine Vielzahl von einzelnen Windeln umfaßt, so daß jeweils zwei Randabschnitte der jeweiligen dritten endlosen Verbundbahnen (64), die sich in Längsrichtung jeweils über zwei beidseits gegenüberliegende Kämme (17a) erstrecken, einander überlappen, und Verkleben der Bahnen (64) entlang den äußeren Seitenrändern mit der Decklage (11) der endlosen Windelbahn; und
E. Schneiden der endlosen Windelbahn mit der nun die beiden dritten endlosen Verbundbahnen (64) verklebt sind, quer entlang Linien (37), die vertikal die Kämme (17a) halbieren, um die einzelnen Windeln zu erhalten.

2. Verfahren nach Anspruch 1,
wobei jeweils zwei Randbereiche der jeweiligen dritten endlosen Verbundbahnen (64), die sich in Längsrichtung über jedes Paar von beidseits gegenüberliegenden Wellenkämmen (17a) erstrecken, in einander überlappender Beziehung miteinander verklebt werden.

## Claims

1. A method of making a skin-contacting top sheet (14) for a disposable nappy with an elasticated opening (16), comprising the steps of:
A. Feeding a continuous elastic element (17) held under tension on to a continuous band in the longitudinal direction thereof, so that the continuous elastic element (17) describes a line running in waves and which is alternately curved towards a first lateral edge (30) and then towards a second lateral edge (31) of the continuous band lying opposite the first edge (30), in order to form wave crests (17a) and relatively wide wave troughs (17b), and bonding the continuous elastic element (17) to the band using an adhesive, in order to obtain a first continuous bonded band (44);
B. Folding a region of the first continuous composite band (44) which extends in the immediate vicinity of the first lateral edge (31) thereof along a fold line (33) running in the longitudinal direction at a level between the first lateral edge (31) and the valleys (17b), so that the respective crests (17a) are included at least partially inside a region between the fold line (33) and the first lateral edge (31), followed by bonding the folded region (32) to the non-folded region (34) of the first continuous composite band, in order to obtain a second continuous composite band (54) which comprises the continuous elastic element (17), which is covered by the continuous band;
C. Cutting the regions (35) of the second continuous composite band (54) which are surrounded by the respective crests (17a), troughs (17b) and the fold line (33), in order to obtain a third continuous composite band (64) which is thus formed with cut-outs (36) which are adapted to delimit the halves of the respective openings (16);
D. Placing together two such third composite bands (64) with each two corresponding cut-outs (36) being aligned opposite one another in a transversely symmetrical relationship, laying these bands (64) on a top sheet (11) of a continuous nappy band which comprises a plurality of individual nappies, so that each two edge sections of the respective third continuous composite bands (64) which extend in the longitudinal direction over two crests (17a) opposite one another on the two sides overlap one another, and bonding the bands (64) along the outer lateral edges to the top sheet (11) of the continuous nappy band; and
E. Cutting the continuous nappy band, to which the two third composite bands (64) are now bonded, transversely along lines (37) which vertically halve the crests (17a), in order to obtain the individual nappies.

2. A method according to claim 1, wherein each two edge regions of the respective third continuous composite band (64) which extend in the longitudinal direction over each pair of wave crests (17a) opposite one another on the two sides are bonded together in overlapping relationship.

## Revendications

1. Procédé pour munir une couche de couverture (14) d'un lange jetable, en contact avec la peau, d'une ouverture élastique (16), comprenant les opérations consistant :
A.- à amener un élément élastique continu (17), maintenu à l'état étiré, sur une bande continue, dans le sens de la longueur de cette dernière, de façon à ce que l'élément élastique continu (17) décrive une ligne ondulée s'incurvant, en alternance, vers un premier bord latéral (30), puis vers un second bord latéral (31) de la bande continue, opposé au premier bord (30), de manière à former, respectivement, des crêtes d'ondulations (17a), et des creux d'ondulations (17b) relativement larges, et à coller l'élément élastique continu (17) à la bande, en utilisant un adhésif, pour obtenir une première bande composite continue (44);
B.- à plier une zone de la première bande composite continue (44) se trouvant à proximité immédiate du premier bord latéral (30) de cette bande, en suivant une ligne de pliage (33) passant longitudinalement, à une certaine hauteur, entre le premier bord latéral (30) et les creux (17b), en sorte que les diverses crêtes (17a) soient comprises, au moins en partie, à l'intérieur d'une zone définie entre la ligne de pliage (33) et le premier bord latéral (30), opération suivie du collage de la zone pliée (32) à la zone non pliée (34) de la première bande composite continue, pour obtenir une deuxième bande composite continue (54) comprenant l'élément élastique (17) recouvert par la bande continue;
C.- à supprimer, en les coupant, les zones (35) de la deuxième bande composite continue (54) délimitées par les crêtes (17a), les creux (17b) et la ligne de pliage (33), pour obtenir une troisième bande composite continue (64), laquelle est ainsi pourvue d'échancrures (36) destinées à délimiter la moitié des ouvertures respectives (16);
D.- à juxtaposer deux troisièmes bandes composites continues (64) du même type, en orientant chaque fois deux échancrures correspondantes (36) en vis-à-vis et alignées mutuellement, dans une relation symétrique dans le sens transversal, à poser ces bandes (64) sur une couche de couverture (11) d'une bande de lange continue comprenant une multitude de langes individuels, de façon à faire se chevaucher deux zones de bordure des troisièmes bandes composites continues (64), zones de bordure qui s'étendent chacune, en sens longitudinal, par-dessus deux crêtes (17a) opposées de part et d'autre, et à coller les bandes (64) à la couche de couverture (11) de la bande de langes continue, en suivant les bords latéraux extérieurs; et
E.- à couper la bande de (anges continue, à laquelle sont à présent collées les deux troisièmes bandes continues (64), transversalement, en suivant des lignes (37) divisant les crêtes (17a) verticalement par moitié, pour obtenir les langes individuels.

2. Procédé selon la revendication 1, selon lequel deux zones de bordure de chacune des troisièmes bandes composites continues (64), qui s'étendent longitudinalement par-dessus chaque paire de crêtes d'ondulations (17a) opposées de part et d'autre, sont collées l'une à l'autre dans une relation de chevauchement mutuel.
